# EUROPEAN PATENT APPLICATION

(11) **EP 1 464 652 A1**
(43) Date of publication of application: **06.10.2004**
(21) Application number: 03290831.1
(22) Date of filing: 02.04.2003
(51) Int. Cl.: C07K 14/47, C07K 14/72

(54) **GPR54 receptor agonist and antagonist useful for the treatment of gonadotropin related diseases**

(71) Applicant: INSTITUT NATIONAL DE LA SANTE ET DE LA RECHERCHE MEDICALE (INSERM), 75654 Paris Cédex 13 (FR); UNIVERSITE PARIS-SUD XI, 91405 Orsay cedex (FR); UNIVERSITE RENE DESCARTES PARIS V, 75270 Paris (FR)
(72) Inventor: De Roux, Nicolas, 92270 Bois-Colombes (FR); Genin, Emmanuelle, 75014 Paris (FR); Milgrom, Edwin, 92230 Sceaux (FR); Chaussain, Jean-Louis, 75007 Paris (FR); Carel, Jean-Claude, 92200 Neuilly sur Seine (FR)
(74) Representative: Cabinet Hirsch

(57) **Abstract**

The invention provides an agonist or antagonist of the GPR54 receptor for its use for treating a gonadotropin related disorder; a ligand of the GPR54 receptor for its use for diagnosing a subject's gonadotropin abnormality; a method for screening a compound that affect the gonadotropic axis comprising the step of assaying the compound in the presence of a GPR54 receptor and novel proteins useful in the above.

SEQUENCE LISTING

<110> INSERM

<120> RECEPTOR AGONIST AND ANTAGONIST USEFUL FOR THE
      TREATMENT OF GONADOTROPIN RELATED DISEASES

<130> 20990EP

<140>
<141>

<160> 2

<170> PatentIn Ver. 2.1

<210> 1
<211> 1197
<212> DNA
<213> Homo sapiens

<220>
<221> CDS
<222> (1)..(1197)

<220>
<221> gene
<222> (1)..(1197)
<223> GPR54 gene

<400> 1
atg cac acc gtg gct acg tcc gga ccc aac gcg tcc tgg ggg gca ccg    48
gcc aac gcc tcc ggc tgc ccg ggc tgt ggc gcc aac gcc tcg gac ggc    96
cca gtc cct tcg ccg cgg gcc gtg gac gcc tgg ctc gtg ccg ctc ttc    144
ttc gcg gcg ctg atg ctg ctg ggc ctg gtg ggg aac tcg ctg gtc atc    192
tac gtc atc tgc cgc cac aag ccg atg cgg acc gtg acc aac ttc tac    240
atc gcc aac ctg gcg gcc acg gac gtg acc ttc ctc ctg tgc tgc gtc    288
ccc ttc acg gcc ctg ctg tac ccg ctg ccc ggc tgg gtg ctg ggc gac    336
ttc atg tgc aag ttc gtc aac tac atc cag cag gtc tcg gtg cag gcc    384
acg tgt gcc act ctg acc gcc atg agt gtg gac cgc tgg tac gtg acg 432
gtg ttc ccg ttg cgc gcc ctg cac cgc cgc acg ccc cgc ctg gcg ctg 480
gct gtc agc ctc agc atc tgg gta ggc tct gcg gcg gtg tct gcg ccg 528
gtg ctc gcc ctg cac cgc ctg tca ccc ggg ccg cgc gcc tac tgc agt 576
gag gcc ttc ccc agc cgc gcc ctg gag cgc gcc ttc gca ctg tac aac 624
ctg ctg gcg ctg tac ctg ctg ccg ctg ctc gcc acc tgc gcc tgc tat 672
gcg gcc atg ctg cgc cac ctg ggc cgg gtc gcc gtg cgc ccc gcg ccc 720
gcc gat agc gcc ctg cag ggg cag gtg ctg gca gag cgc gca ggc gcc 768
gtg cgg gcc aag gtc tcg cgg ctg gtg gcg gcc gtg gtc ctg ctc ttc 816
gcc gcc tgc tgg ggc ccc atc cag ctg ttc ctg gtg ctg cag gcg ctg 864
ggc ccc gcg ggc tcc tgg cac cca cgc agc tac gcc gcc tac gcg ctt 912
aag acc tgg gct cac tgc atg tcc tac agc aac tcc gcg ctg aac ccg 960
ctg ctc tac gcc ttc ctg ggc tcg cac ttc cga cag gcc ttc cgc cgc 1008
gtc tgc ccc tgc gcg ccg cgc cgc ccc cgc cgc ccc cgc cgg ccc gga 1056
ccc tcg gac ccc gca gcc cca cac gcg gag ctg ctc cgc ctg ggg tcc 1104
cac ccg gcc ccc gcc agg gcg cag aag cca ggg agc agt ggg ctg gcc     1152
gcg cgc ggg ctg tgc gtc ctg ggg gag gac aac gcc cct ctc tga         1197

<210> 2
<211> 399
<212> PRT
<213> Homo sapiens

<400> 2

SEQUENCE LISTING

<110>  INSERM

<120>  RECEPTOR AGONIST AND ANTAGONIST USEFUL FOR THE TREATMENT OF GONADOTROPIN
RELATED DISEASES

<130>  20990EP

<160>  2

<170>  PatentIn version 3.1

<210>  1
<211>  1197
<212>  DNA
<213>  Homo sapiens

<220>
<221>  CDS
<222>  (1)..(1197)
<223>

<220>
<221>  gene
<222>  (1)..(1197)
<223>  GPR54 gene

<400>  1
atg cac acc gtg gct acg tcc gga ccc aac gcg tcc tgg ggg gca ccg        48
gcc aac gcc tcc ggc tgc ccg ggc tgt ggc gcc aac gcc tcg gac ggc        96
cca gtc cct tcg ccg cgg gcc gtg gac gcc tgg ctc gtg ccg ctc ttc       144
ttc gcg gcg ctg atg ctg ctg ggc ctg gtg ggg aac tcg ctg gtc atc       192
tac gtc atc tgc cgc cac aag ccg atg cgg acc gtg acc aac ttc tac          240
atc gcc aac ctg gcg gcc acg gac gtg acc ttc ctc ctg tgc tgc gtc          288
ccc ttc acg gcc ctg ctg tac ccg ctg ccc ggc tgg gtg ctg ggc gac          336
ttc atg tgc aag ttc gtc aac tac atc cag cag gtc tcg gtg cag gcc          384
acg tgt gcc act ctg acc gcc atg agt gtg gac cgc tgg tac gtg acg          432
gtg ttc ccg ttg cgc gcc ctg cac cgc cgc acg ccc cgc ctg gcg ctg          480
gct gtc agc ctc agc atc tgg gta ggc tct gcg gcg gtg tct gcg ccg          528
gtg ctc gcc ctg cac cgc ctg tca ccc ggg ccg cgc gcc tac tgc agt          576
gag gcc ttc ccc agc cgc gcc ctg gag cgc gcc ttc gca ctg tac aac          624
ctg ctg gcg ctg tac ctg ctg ccg ctg ctc gcc acc tgc gcc tgc tat          672
gcg gcc atg ctg cgc cac ctg ggc cgg gtc gcc gtg cgc ccc gcg ccc          720
gcc gat agc gcc ctg cag ggg cag gtg ctg gca gag cgc gca ggc gcc          768
gtg cgg gcc aag gtc tcg cgg ctg gtg gcg gcc gtg gtc ctg ctc ttc          816
gcc gcc tgc tgg ggc ccc atc cag ctg ttc ctg gtg ctg cag gcg ctg          864
ggc ccc gcg ggc tcc tgg cac cca cgc agc tac gcc gcc tac gcg ctt          912
aag acc tgg gct cac tgc atg tcc tac agc aac tcc gcg ctg aac ccg          960
ctg ctc tac gcc ttc ctg ggc tcg cac ttc cga cag gcc ttc cgc cgc          1008
gtc tgc ccc tgc gcg ccg cgc cgc ccc cgc cgc ccc cgc cgg ccc gga     1056
ccc tcg gac ccc gca gcc cca cac gcg gag ctg ctc cgc ctg ggg tcc     1104
cac ccg gcc ccc gcc agg gcg cag aag cca ggg agc agt ggg ctg gcc     1152
gcg cgc ggg ctg tgc gtc ctg ggg gag gac aac gcc cct ctc tga         1197

<210>  2

<211>  398

<212>  PRT

<213>  Homo sapiens

<400>  2

## Description

### FIELD OF THE INVENTION.

The invention relates to GPR54 receptor agonist and antagonist that are useful for the treatment of gonadotropin related diseases, as well as in the diagnostic field. These compounds will find application in many pathologies, known to be dependant upon GnRH or LH/FSH secretion.

### BACKGROUND OF THE INVENTION.

The integrity of the gonadotropic axis leads to normal sexual differentiation during foetal life, normal puberty and therefore to normal fertility. Genetic defects leading to isolated impuberism and infertility have been described in genes encoding for known proteins as GnRH receptor, gonadotropins, gonadotropin receptors and steroidogenic enzymes.

Hypogonadisms related to deficiency of GnRH or LH/FSH synthesis are called hypogonadotropic hypogonadism. Cases of congenital isolated hypogonadotropic hypogonadism are classified into 2 categories: those associated with anosmia (Kallmann syndrome) and those apparently isolated. Mutations and deletions of the KAL-1 gene have been observed in many cases of the X-linked form of Kallmann syndrome. Cases of hypogonadotropic hypogonadism without anosmia were considered as idiopathic until the recent description of mutations of the GnRH receptor gene.

Inhibition or activation of the gonadotropic axis are useful schemes for the treatment of hormones-related diseases, such as gonadotropin deficiency, precocious puberty, and some types of cancer (e.g. prostate and breast cancers) and useful to manage in-vitro fecundation. For the time being, only the GnRH receptor is known to play a role in regulating LH and FSH secretion, although it is possible that GnRH receptor may itself has regulation effects in cancer independent of LH and FSH.

GPR54 was initially described as an orphan receptor homolog to galanin receptor. Recently, a ligand acting on GPR54 bas been described. Katani et al as well as Ohtaki et al analysed placental extracts for peptides activating GPR54. Muir et al used a library of 1500 putative ligands. The best agonists displayed a similarity to a 54 amino acids peptide derived from the KiSS-*1* protein (also found were peptides of 14, 13 and even fewer amino acids). This peptide corresponds to the predicted proteolytic processing of Kiss-*1* at dibasic and dibasic/amidation sites, and have been named Kisspeptins. GPR54 stimulation by this 54 amino acid peptide results in the activation of phospholipase C by coupling to a Gq protein. It was also determined that GPR54 was mainly present in pituitary and placenta, and that Kisspeptins are high affinity agonists of the GPR54 receptor. Kotani et al. concludes that tissue distribution suggested that GPR54 might be implicated in various hormonal functions, a hypothesis supported by the demonstration that KISS-1 derived peptides stimulate oxytocin release in rats.

Human and rat GPR54 genes have been fully disclosed in many publications, referred to in the above Kotani, Ohtaki and Muir publications. *GPR54* gene is formed of 5 exons. GPR54 has also sometimes been named AXOR12 or SNORF11.

WO-A-2003003983 discloses a method of treating an abnormality which comprises administering to the subject an amount of a SNORF11 (GPR54) receptor agonist. Examples of such agonists that are given include KISS-1 peptide fragments. It is also indicated that the SNORF11 (GPR54) receptor may serve as a tool for designing drugs for treating various pathological conditions, including, cancers, sexual/reproductive disorders, benign prostatic hypertrophy. The sole example given relates to pain.

US-A-20020106766 discloses the rat AXOR12 gene sequence and potential uses of agonists and antagonists of the receptor. There is however no working example of any pathology in this document.

EP-A-1126028 (naming Ohtaki as an inventor) discloses GPR54 gene, encoded protein, ligands and potential uses, which include diagnostic and screening uses. Rat and human GPR54 proteins and coding sequences are disclosed in SEQ ID NO:1 and 5, respectively.

However, none of the above documents teaches or suggests the present invention.

### SUMMARY OF THE INVENTION.

The invention shows that GPR54 is a new hormonal system playing an important and previously unsuspected role in the physiology of the gonadotropic axis.

Hence, the invention offers a further route for defining new pharmacological strategies to activate or inhibit the gonadotropic axis and investigating gonadotropic hormones related pathologies.

The invention thus provides an agonist or antagonist of the GPR54 receptor for its use for treating a gonadotropin related disorder.

In one embodiment, the GPR54 receptor is the protein shown in SEQ ID NO:2, or a partial protein thereof, or an ester, amide or salt thereof.

In another embodiment, the GPR54 receptor is the protein shown in SEQ ID NO:2 from amino-acids 247 to 398.

In another embodiment, the GPR54 receptor is the protein shown in SEQ ID NO:2 with the mutation L102P.

The agonist or antagonist of the invention is useful for its use for treating hypogonadotropic hypogonadism, LH and/or FSH related disorders, gonadotropin-estradiol/testosterone-dependent related cancers.

The invention also provides a ligand of the GPR54 receptor for its use for diagnosing a subject's gonadotropin abnormality, such as hypogonadotropic hypogonadism.

In one embodiment, the ligand of the invention binds to the protein shown in SEQ ID NO:2 from amino-acids 247 to 398.

In another embodiment, the ligand of the invention binds to the protein shown in SEQ ID NO:2 with the mutation L102P.

The invention also provides a method for screening a compound that affect the gonadotropic axis comprising the step of assaying the compound in the presence of a GPR54 receptor.

In one embodiment, the screening method of the invention aims at screening for a compound that effects the LH and/or FSH secretion.

In one embodiment, in the screening method of the invention, the GPR54 receptor is the protein shown in SEQ ID NO:2, or a partial protein thereof, or an ester, amide or salt thereof.

In another embodiment, in the screening method of the invention, the GPR54 receptor is the protein shown in SEQ ID NO:2 from amino-acids 247 to 398.

In another embodiment, in the screening method of the invention, the GPR54 receptor is the protein shown in SEQ ID NO:2 with the mutation L102P.

Finally, the invention provides novel proteins, i.e. the protein shown in SEQ ID NO:2 from amino-acids 247 to 398 as well as the protein shown in SEQ ID NO:2 with the mutation L102P.

### BRIEF DESCRIPTION OF THE FIGURES AND SEQUENCES.

Figure 1 shows the pedigrees of the two affected families.
Figure 2 is a GnRH (100 µg/iv) test performed in the propositus of family 2
Figure 3 is the amino acid sequence of human *GPR54.* Boxes highlight putative transmembrane domains. The site of the deletion observed in affected individuals is indicated by an arrow. The deleted protein sequence is in italics.

SEQ ID NO:1 is the sequence of human GPR54 (gene and encoded protein) while SEQ ID NO:2 is the sequence of the protein.

### DETAILED DESCRIPTION OF THE INVENTION.

As indicated above, the inventors have found that GPR54 plays an important and previously unsuspected role in the physiology of the gonadotropic axis. The present invention describes a new genetic etiology for impuberism. It shows that alteration of GPR54 (KiSS-1 peptide receptor) plays an important and previously unsuspected role in the initiation of puberty. Therefore, loss of function of GPR54 leads to hypogonadotropic hypogonadism.

This was demonstrated by sequencing the GPR54 nucleotide sequence of affected patients, where the patients were suffering from hypogonadotropic hypogonadism (impuberism). GPR54 was chosen as candidate gene as it is localized in the region of interest defined by genome mapping in a very informative family. A homozygous deletion within intron 4 and exon 5 of the GPR54 gene was found in all affected siblings in one family. In a second family showing a recessive transmission, a homozygous point mutation was found within exon 1.

These findings will have useful applications in diagnostic and drug design, in pathologies that are related to GnRH or gonadotropin secretion.

The GPR54 receptor proteins and the like are useful, among other things: (1) for determination of an agonist or antagonist to the GPR54 receptor, where these agonist and antagonist compounds would be useful in gonadotropin-related diseases, (2) for screening of compounds (agonist, antagonist, etc.) that alter the binding property between GPR54 and a ligand, whereby the screened compound would then be useful for the treatment of gonadotropin-related diseases and (3) for diagnosing gonadotropin-related diseases, as a genetic diagnostic agent or (4) for determination of a compound leading to perform dynamic hormonal tests of the gonadotropic axis during diagnosis procedure.

Gonadotropin-related diseases include those pathologies involving malfunction in the LH and/or FSH secretion, hypogonadotropic hypogonadism, precocious puberty, uterine leiomyomas), severe endometriosis, hyperandrogenism, menometrorrhagia, catamenial disorders and endometrial hyperplasia, and prostate and breast cancers known to be LH-dependent estradiol/testosterone-dependent disorders.

The screening methods of the invention can be carried out according to known methods. Those depicted in EP-A-1126028, WO-A-2003003983 and US-A-20020106766 are suitable.

The screening method may measure the binding of a candidate compound to the receptor, or to cells or membranes bearing the receptor, or a fusion protein thereof by means of a label directly or indirectly associated with the candidate compound. Alternatively, a screening method may involve measuring or, qualitatively or quantitatively, detecting the competition of binding of a candidate compound to the receptor with a labelled competitor (e.g., agonist or antagonist). Further, screening methods may test whether the candidate compound results in a signal generated by an agonist or antagonist of the receptor, using detection systems appropriate to cells bearing the receptor. Antagonists can be assayed in the presence of a known agonist and an effect on activation by the agonist by the presence of the candidate compound is observed. Further, screening methods may comprise the steps of mixing a candidate compound with a solution comprising a GPR54 receptor, to form a mixture, and measuring the activity in the mixture, and comparing to a control mixture which contains no candidate compound. Competitive binding using known peptide agonist such as the KISS peptides mentioned above is also suitable.

Assays techniques are known in the art and the skilled man may revert to publications to that effect, such as the mentioned patents, e.g. EP-A-1126028, WO-A-2003003983 and US-A-20020106766.

The GPR54 receptor of the present invention may be employed in conventional low capacity screening methods and also in high-throughput screening (HTS) formats.

Screening kits can then be manufactured using known techniques.

Once screened and identified, the useful compounds are conventionally used as pharmaceutical compositions.

The diagnostic methods may be carried out using the methods disclosed in EP-A-1126028. Notably, antibodies can be used, where the antibodies, monoclonal or polyclonal can be manufactures by publicly known methods. Other ligands can be used, as long as they allow recognition the presence (or absence) of (part) of the GPR54 protein.

Diagnostic kits can then be manufactured using known techniques.

The GPR54 protein useful in the present invention is one that has an amino sequence identical or substantially similar to the one depicted in SEQ ID NO:2. Preferably, the sequence includes an amino acid sequence having at least about 70% homology, preferably at least about 80% homology, more preferably at least about 90% homology, most preferably at least about 95% homology, to the protein sequence represented by SEQ ID NO:2. Partial peptides can be used.

The instant invention is not limited to human GPR54, but can be applied to any other mammals, including those useful in the agricultural field, it being understood that the GPR54 is the one corresponding to the mammal of interest.

Specific examples include the protein corresponding to the polypeptide from residue 247 to 398 of SEQ ID NO:2 (hereinafter deleted or truncated GPR54 protein) or the polypeptide shown in SEQ ID NO:2 with the mutation L102P (proline substituted for leucine) (hereinafter 102-mutated GPR54 protein)

The receptor protein of the present invention which can be used may be a protein comprising (i) an amino acid sequence represented by SEQ ID NO:2, or the truncated or 102-deleted corresponding protein, in which one, two, or more amino acids (preferably 1 to 30 amino acids, more preferably 1 to 10 amino acids, most preferably 1 or 2 amino acids) are deleted; (ii) an amino acid sequence represented by SEQ ID NO:2, or the truncated or 102-deleted corresponding protein, to which one, two, or more amino acids (preferably 1 to 30 amino acids, more preferably 1 to 10 amino acids, most preferably 1 or 2 amino acids) are added; (iii) an amino acid sequence represented by SEQ ID NO:2, or the truncated or 102-deleted corresponding protein, in which one, two, or more amino acids (preferably 1 to 30 amino acids, more preferably 1 to 10 amino acids, and most preferably 1 or 2 amino acids) are substituted by other amino acids; and (iv) a combination of the above amino acid sequences.

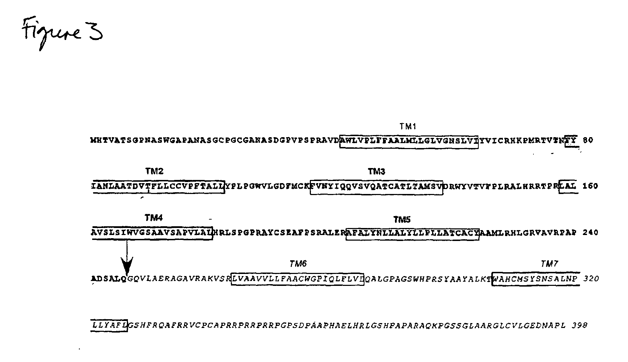

The partial peptide of the GPR54 receptor protein of the present invention (hereinafter sometimes referred to as the partial peptide) may be any partial peptide, so long as it constitutes a part of the peptide portions of the receptor protein described above retaining binding properties. Examples of such partial peptides include site, which is exposed outside cell membranes among the receptor protein and retain the receptor binding activity or the transmembrane domains. These domains are identified in figure 3.

An example is a peptide containing a region which is analyzed to be an extracellular area (hydrophilic region or site) in a hydrophobic plotting analysis.

It is also possible to have partial peptides fused together.

The number of amino acids in the partial peptide of the present invention is at least 20 or more, preferably 50 or more, more preferably 100 or more, in terms of the constructive amino acid sequence of the GPR54 receptor protein described above.

Esters, amides or salts can also be used, as disclosed in EP-A-1126028.

The receptor protein of the present invention may be manufactured in accordance with a publicly known method for purification of a receptor protein from human or other mammalian cells or tissues. Alternatively, the receptor protein of the present invention or salts thereof may also be manufactured by culturing a transformant containing DNA encoding the receptor protein of the present invention, as will be later described. Furthermore the receptor protein of the present invention or salts thereof may also be manufactured by known methods for synthesizing proteins.

Finally, the invention provides two specific proteins, one being truncated or deleted, and the other being mutated. The invention also provides the polynucleotides (purified) encoding said proteins, a vector comprising said polynucleotide and a host cell comprising the vector.

Also within the ambit of the invention is the antisense nucleic acid, as well as the gene therapy using the above GPR54 receptor.

The G protein coupled receptor may be used not only for administration of antagonists or agonists of the receptor, but also for gene therapy by transfer of the receptor gene into the body (or certain specific organs such as the hypophysis) or by transfer of the antisense nucleic acid to the receptor gene.

Antisense nucleic acids that can inhibit replication or expression of the GPR54 receptor protein gene can inhibit RNA synthesis or the function of RNA, or can regulate/control the expression of the receptor protein gene via the interaction with RNAs associated with the receptor protein. Antisense nucleic acids are useful for regulating and controlling the expression of the receptor protein gene in vivo and in vitro, and are also useful for the treatment and diagnosis of the diseases disclosed above.

Technologies related to such antisense RNAs and gene therapies are known to the skilled man.

### EXAMPLES.

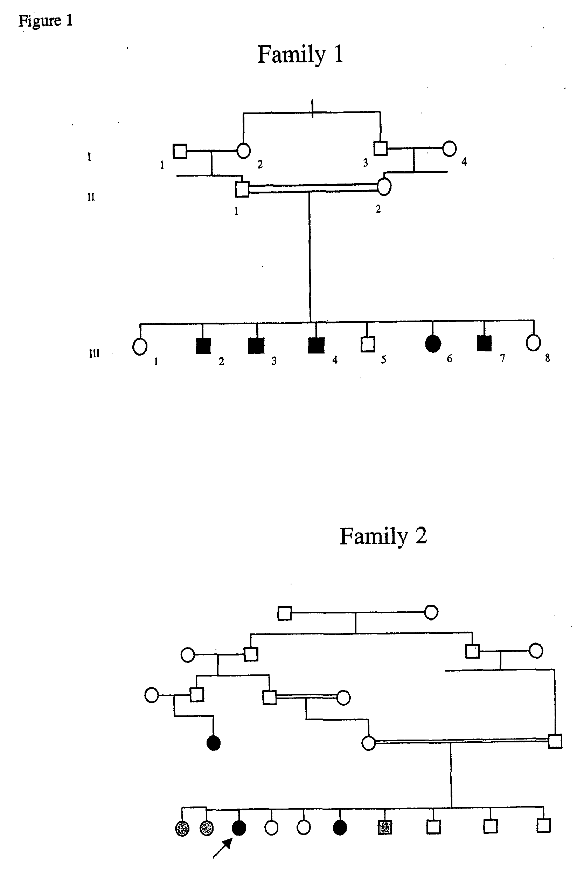

A consanguineous family (family 1) with 5 affected sibs was investigated (see Fig. 1). The propositus was a 20-year old male referred for impuberism. He had typical signs of hypogonadism with small testes (28x17 mm), sparse pubic hair (P3) and a penis of 7 cm. His bone age was retarded at 15.0 years. He had a normal sense of smell and showed no abnormal eye movements, no colour blindness and no renal or cranio-facial abnormalities. His weight and height were 54 kgs and 152 cm respectively. Three brothers presented similar clinical signs. A sister had a partial hypogonadism. At 16, she had partial breast development and she reported a single episode of uterine bleeding. Hormone assays (Table 1) showed low plasma testosterone in boys and low plasma oestradiol in the sister accompanied by low plasma gonadotropin levels. All sibs had a partial or a blunted response to GnRH (100µg IV). One other brother and two other sisters had a normal pubertal development. The parents were first cousins and have had normal pubertal development. Table 1 below gives the hormonal status of the affected patients of family 1

| Patient | Age | Bone age | Plasma Testosterone (ng/dl) | Plasma Oestradiol (pg/ml) | Plasma LH (mU/ml) | Plasma FSH (mU/ml) | GnRH test | |
|---|---|---|---|---|---|---|---|---|
| | | | | | | | LH | FSH |
| III.2 | 21 | 15 | 26 | - | 1.5 | 0.5 | 3.6 | 1.7 |
| III.3 | 20 | 15 | 19 | - | 1.5 | 0.5 | 1.4 | 1.5 |
| III.4 | 19 | - | 5 | - | 1.1 | 4.1 | 1.9 | 4.1 |
| III.6 | 18 | - | - | 17 | 2.0 | 3.4 | 11.8 | 6.4 |
| III.7 | 14 | 11 | 5 | - | 2.6 | 1.8 | 3.4 | 2.6 |

The chronological age and the bone age are indicated. Normal values: (males) LH, 1.0-5.0 IU/ml, FSH 0.9-5.7 IU/ml), Testosterone 260-690 ng/dl; (Females) LH, 1.1-5.4 IU/ml, FSH 2.3-6.0 IU/ml, Oestradiol (early follicular phase) 25-90 pg/ml. The GnRH test was performed by intra venous administration of 100 µg of GnRH. The highest values observed for plasma LH and FSH are reported.

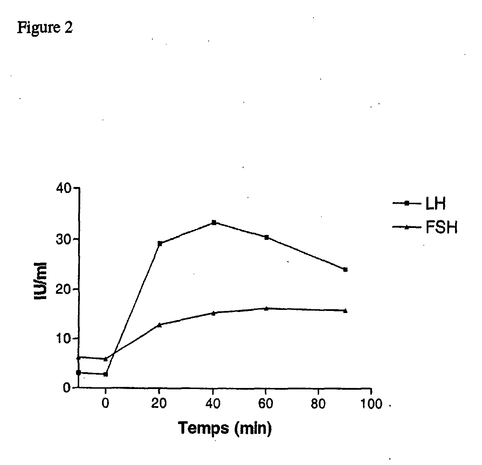

The second family (family 2) was a consanguineous family originated from Kurdistan. The propositus was a 27 years old woman referred for primary amenorrhea. She had a normal breast and pubic hair development. She had a normal sense of smell. Ultrasonography showed a small uterus with thin infantile endometrium. Ovaries were small with several small immature follicles. Plasma oestradiol is low accompanied by normal plasma gonadotropins. All other anterior pituitary hormone plasma levels were in normal range. The GnRH test performed with 100 µg/IV showed a normal response for the FSH and an explosive response for LH (see figure 2). LH pulsatility showed low amplitude peaks but normal frequency. A pulsatile GnRH pump administration led to a normal pregnancy.

For both family sibs, genomic DNA was isolated from peripheral lymphocytes following standard methods.

The 5 exons of the *GPR54* gene were amplified by PCR with 20 to 100 ng of genomic DNA. The following primers were used:

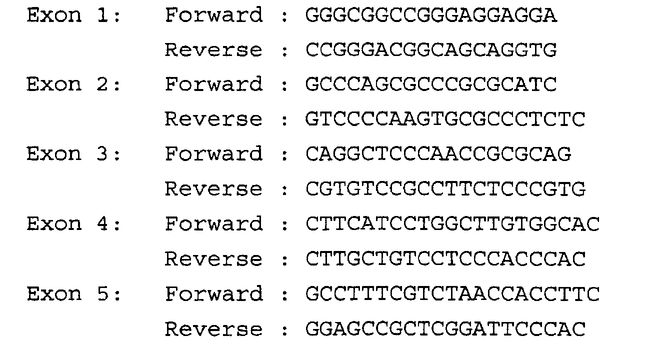

Amplification was performed for 30 cycles with Yellow Taq (Eurogentec) in 1.5 5 mM MgCl₂, with 0.1 µM of each primer and 5% DMSO. The annealing temperatures were of 60° for exons 1, 3, 4, 5 and of 66° for exon 2. The PCR products were directly sequenced with BigDye dideoxyterminator cycle sequencing kits and the 3100 sequencer (Applied Biosystems) using the same primers. To genotype all members of the family, the PCR products of exon 5 were analyzed by electrophoresis in 2% agarose gel.

Upon study of the *GPR54* gene, it was observed in affected individuals a homozygous deletion of 155 base pairs lying between intron 4 (nucleotide -13 when numbering from the 3' end of the intron 4) and exon 5 (nucleotide 142 of the exon 5, corresponding to nucleotide 880 of the cDNA). The deletion reported in family 1 removes the splicing acceptor site of intron 4-exon 5 junction. It thus leads to the absence of the normal protein sequence downstream from residue 247 (Fig. 3). The deleted receptor is truncated within the third intracellular loop thus lacking transmembrane domains 6 and 7 (Fig. 3). All affected patients were homozygous for this deletion. Both parents as well as unaffected sib III.5 were heterozygous. Unaffected sib III.1 was homozygous for the wild type sequence. The deletion was absent in 50 control subjects.

In family 2 showing a recessive transmission, a homozygous point mutation was found within exon 1. This mutation substituted a proline for a leucine (L102P) at the N-terminal extremity of the first extracellular loop.

## Claims

1. An agonist or antagonist of the GPR54 receptor for its use for treating a gonadotropin related disorder.

2. The agonist or antagonist of claim 1, where the GPR54 receptor is the protein shown in SEQ ID NO:2, or a partial protein thereof, or an ester, amide or salt thereof.

3. The agonist or antagonist of claim 1 or 2, where the GPR54 receptor is the protein shown in SEQ ID NO:2 from amino-acids 247 to 398.

4. The agonist or antagonist of claim 1 or 2, where the GPR54 receptor is the protein shown in SEQ ID NO:2 with the mutation L102P.

5. The agonist or antagonist of any one of claims 1 to 4 for its use for treating hypogonadotropic hypogonadism.

6. The agonist or antagonist of any one of claims 1 to 4 for its use for treating LH and/or FSH related disorders.

7. The agonist or antagonist of any one of claims 1 to 4 for its use for treating gonadotropin-estradiol/testosterone-dependent related cancers.

8. A ligand of the GPR54 receptor for its use for diagnosing a subject's gonadotropin abnormality.

9. The ligand of claim 8 for its use for diagnosing hypogonadotropic hypogonadism.

10. The ligand of claim 9 that binds to the protein shown in SEQ ID NO:2 from amino-acids 247 to 398.

11. The ligand of claim 9 that binds to the protein shown in SEQ ID NO:2 with the mutation L102P.

12. A method for screening a compound that affect the gonadotropic axis comprising the step of assaying the compound in the presence of a GPR54 receptor.

13. The method of claim 12, for screening for a compound that effects the LH and/or FSH secretion.

14. The method of claim 12 or 13, in which the GPR54 receptor is the protein shown in SEQ ID NO:2, or a partial protein thereof, or an ester, amide or salt thereof.

15. The method of any one of claims 12 to 14, where the GPR54 receptor is the protein shown in SEQ ID NO:2 from amino-acids 247 to 398.

16. The method of any one of claims 12 to 14, where the GPR54 receptor is the protein shown in SEQ ID NO:2 with the mutation L102P.

17. A protein shown in SEQ ID NO:2 from amino-acids 247 to 398.

18. A protein shown in SEQ ID NO:2 with the mutation L102P.
